# EUROPÄISCHE PATENTSCHRIFT

(11) **EP 1 103 260 B1**
(45) Veröffentlichungstag und Bekanntmachung des Hinweises auf die Patenterteilung: **26.01.2005**
(21) Anmeldenummer: 00126102.3
(22) Anmeldetag: 29.11.2000
(51) Int. Cl.: A61K 31/315, A61K 9/70, A61K 31/415

(54) **Transdermalsystem zur Abgabe von Clonidin**
Transdermal system for the administration of clonidine
Système transdermique pour l'administration de clonidine

(30) Priorität: 29.11.1999 US 451180
(43) Veröffentlichungstag der Anmeldung: 30.05.2001
(73) Patentinhaber: Novosis AG, 83714 Miesbach (DE)
(72) Erfinder: Fischer, Wilfreid, 80807 München (DE); Huber, Petra, 80807 München (DE); Bostedt, Katalin, Tisa, 80807 München (DE)

(56) Entgegenhaltungen:
- EP-A- 0 887 075
- WO-A-99/02141
- CHEMICAL ABSTRACTS, vol. 110, no. 12, 20. März 1989 (1989-03-20) Columbus, Ohio, US; abstract no. 101838, NAKAGAWA, AKIRA ET AL: "Sustained-release pharmaceutical transdermal tapes containing clonidine" XP002169756 & JP 63 203616 A (HISAMITSU PHARMACEUTICAL CO., INC., JAPAN) 23. August 1988 (1988-08-23)

## Beschreibung

Die Erfindung betrifft wirkstoffhaltige Transdermalsysteme (im folgenden auch Matrixpflaster oder einfach Pflaster genannt) zur Abgabe von Clonidin und ihre Verwendung zur Behandlung von Hypertonie, Migräne, Angstzuständen, hyperkinetischen Verhaltensstörungen, Entzugserscheinungen bei Alkohol- oder Drogenentzug und menopausalen Symptomen.

Wirkstoffhaltige Transdermalsysteme ("Pflaster") sind dem Fachmann auf dem Gebiet der pharmazeutischen Technologie seit ca. 20 Jahren bekannt Im wesentlichen werden zwei große technische Systeme unterschieden: Matrix- und Reservoirsysteme. Die Erfindung betrifft nur Matrixsysteme, bei denen medizinische Wirkstoffe direkt in eine halbfeste Matrix aus Polymeren eingebettet werden.

Clonidin ist ein Antisympathotonikum mit Imidazolin-Struktur. Es weist Affinität zu α1- - und stärker - zu prä- und postsynaptischen α2-Adrenorezeptoren auf und senkt den peripheren Sympathikustonus. Clonidin bewirkt in erster Linie eine Blutdrucksenkung aufgrund absinkenden Herzzeitvolumens und - bei längerer Medikation - durch Verminderung des peripheren Gefäßwiderstandes. Zugleich vermindert es die Renin-Ausschüttung mit einer Abnahme von Angiotensin II im Blutplasma unter Freisetzung von Aldosteron aus der Nebennierenrinde.

Clonidin wird z. B. bei folgenden Indikationen eingesetzt:
- Hypertonie
- Migräne
- Angstzuständen
- hyperkinetischen Verhaltensstörungen
- Entzugserscheinungen bei Alkohol- oder Drogenentzug
- menopausalen Symptomen

Clonidin-Hydrochlorid existiert als mesomerische Komponente. Der chemische Name ist 2-(2,6-Dichlorophenylamino)-2-imidazolin-Hydrochlorid. Molekülformel: C₉H₉Cl₂N₃ · HCl, Molekularmasse: 266.56

Es sind verschiedene Transdermalsysteme, die Clonidin enthalten, entwickelt worden. So beschreibt das US-Patent 4,559,222 vom 17. Dezember 1985 ein mehrschichtiges Transdermalsystem, in dem Clonidin-Base in Mineralöl zusammen mit kolloidalem Siliciumdioxid in einer ersten Schicht in einem Polyisobutylenklebstoff enthalten ist. Auf diese Schicht wird eine mikroporöse Membrane aufgebracht, auf die wiederum eine Klebstoffschicht aufgebracht wird. Diese Klebstoffschicht wird auf die Haut geklebt. Das Transdermalsystem ist auf der Seite der wirkstoffhaltigen Schicht mit einer für Clonidin undurchlässigen Folie abgedeckt. Nachteile dieses Systems sind die bekannte schlechte Hautverträglichkeit von Polyisobutylenklebstoffen, das komplizierte und teure Herstellungsverfahren durch die vielen benötigten Schichten und die prinzipiell auftretende physikalische Instabilität des Systems, da die mit der Haut in Kontakt tretende Schicht sich im Lauf der Lagerung mit Clonidin sättigt, wodurch sich das Freisetzungsverhalten des Systems verändert, d.h. ein länger gelagertes System setzt nach dem Aufkleben auf die Haut den Wirkstoff aus der Kontaktschicht schneller frei als durch die mikroporöse Membrane nachgeliefert werden kann. Ein weiterer Nachteil ist die schlechte Klebkraft des Systems. Da das Transdermalsystem sieben Tage lang getragen werden soll, muß der Hersteller ein wirkstoffreies Pflaster mitliefern, das über das eigentliche Clonidin enthaltende System zur zuverlässigen Fixierung geklebt werden muß. Dies erhöht weiterhin die Kosten sowie den Aufwand des Anwenders.

Das US-Patent 5,762,952 vom 9. Juni 1998 beschreibt ein verbessertes System, bestehend aus einem selbstvernetzenden Acrylatklebstoff, in den z. B. Clonidin zusammen mit bei höheren Temperaturen flüchtigen Hilfssstoffen wie Lösemitteln oder Resorptionsförderern eingearbeitet wird. Die Vernetzung ist notwendig, um die Konsistenz der Klebstoffmasse zu erhöhen, die durch den Zusatz hoher Mengen flüssiger Komponenten wie Lösemittel oder Resorptionsförderer stark soweit vermindert wird, daß keine kohärente Klebstoffschicht mehr entsteht. Nachteile dieser Erfindung sind zum einen die Verwendung toxischer Vernetzer sowie potentiell hautirritierender Lösemittel und Resorptionsförderer.

Das US-Patent 5,958,446 beschreibt eine Erfindung, nach der ein Gemisch aus selbstklebenden Acrylaten und Polyisobutylen oder Silikonen einen höheren Fluß durch die Haut ergibt als bei alleiniger Verwendung der Polymeren. Das Patent beansprucht zwar die Verwendung von Clonidin als Wirkstoff, führt jedoch kein Beispiel dazu aus. Der Nachteil der beschriebenen Erfindung liegt darin, daß die Kombination zweier Polymerer in der überwiegenden Anzahl der beschriebenen Beispiele (z. B. mit 17β-Estradiol, Norethisteronacetat, Pilocarpin, sämtlich Substanzen, die gut durch die Haut penetrieren) unter Anwendung von Resorptionsförderern wie Lecithin oder Propylenglycol hergestellt wurde, um einen ausreichenden Fluß zu erhalten. Das heißt, die Verwendung der im Patent beschriebenen Gemische aus Polymeren allein reicht nicht aus, um Transdermalsysteme mit ausreichender Wirkung herzustellen.

Die Aufgabe der vorliegenden Erfindung liegt nun darin, ein Transdermalsystem zur Abgabe von Clonidin zur Verfügung zu stellen, das sehr kostengünstig produziert werden kann, sehr hautschonend ist, für die Patienten einfach anzuwenden ist, keine zusätzliche Fixierhilfe benötigt, zwischen 100 und 300 µg Clonidin pro Tag durch die Haut freigibt und keine toxischen Vernetzer oder Lösemittel/Resorptionsförderer enthält

Erfindungsgemäß wird diese Aufgabe mit einem Transdermalsystem zur Abgabe von Clonidin gemäß Patentanspruch 1 gelöst.

Die Erfindung betrifft somit Transdermalsysteme zur Abgabe von Clonidin aus einer Clonidin enthaltenden Haftkleberschicht aus einem Styrol-Blook-Copolymer, die keine toxischen Vernetzer oder Lösemittel oder Resorptionsförderer enthält, sowie neben der Clonidin enthaltenden Haftkleberschicht einer Abdeckung und auf der der Abdeckung gegenübertiegenden Seite einem die Haftkleberschicht temporär abdeckenden und abziehbaren Träger.

Die Erfindung betrifft ferner die Verwendung dieser Transdermalsysteme zur Behandlung von Hypertonie, Migräne, Angstzuständen, hyperkinetischen Verhaltensstörungen, Entzugserscheinungen bei Alkohol- oder Drogenentzug und menopausalen Symptomen gemäß Patentanspruch 15.

Weitere vorteilhafte und bevorzugte Ausführungsformen sind Gegenstand der Unteransprüche.

Eine vorteilhafte Ausführungsform ist dadurch gekennzeichnet, daß die Haftkleberschicht Clonidin im Konzentrationsbereich von 0,1 bis 20 Gew.-% aufweist.

Eine weitere vorteilhafte Ausführungsform ist dadurch gekennzeichnet, daß die Haftkleberschicht Clonidin im Konzentrationsbereich von 2 bis 10 Gew.-% aufweist.

Eine weitere vorteilhafte Ausführungsform ist dadurch gekennzeichnet, daß die Clonidin enthaltende Haftkleberschicht eine Schicht eines flächigen selbstklebenden Pflasters mit mehrschichtigem Aufbau bildet.

Eine weitere vorteilhafte Ausführungsform ist dadurch gekennzeichnet, daß die Abdeckung aus Kunststoffolie, Kunststoffschaum, Gewebe oder Vlies besteht.

Eine weitere vorteilhafte Ausführungsform ist dadurch gekennzeichnet, daß der Träger aus Kunststoffolie, Papier oder einem Laminat daraus besteht.

Eine weitere vorteilhafte Ausführungsform ist dadurch gekennzeichnet, daß der Träger silikonisiert ist.

Eine weitere vorteilhafte Ausführungsform ist dadurch gekennzeichnet, daß es sich bei der Kunststoffolie um Polyester-, Polyethylen- oder Polypropylen-Folie handelt.

Eine weitere vorteilhafte Ausführungsform ist dadurch gekennzeichnet, daß die trockene Haftkleberschicht ein Flächengewicht von 20 bis 150 g/m² hat. Eine weitere vorteilhafte Ausführungsform ist dadurch gekennzeichnet, daß die trockene Haftkleberschicht ein Flächengewicht von 50 bis 120 g/m² hat.

Eine weitere vorteilhafte Ausführungsform ist dadurch gekennzeichnet, daß die Abgaberate 10 bis 1000 µg Clonidin pro Tag beträgt.

Eine weitere vorteilhafte Ausführungsform ist dadurch gekennzeichnet, daß die Abgaberate 50 bis 500 µg Clonidin pro Tagbeträgt.

Im folgenden wird die Erfindung ohne Beschränkung detaillierter beschrieben.

Die Herstellung von Clonidin-Pflastern wird auf herkömmlichen Maschinen, die dem Fachmann bekannt sind, vorgenommen.

Clonidin-Base wird in einem geeigneten, leicht flüchtigen Lösemittel, z. B. Ethylacetat, Ethanol oder Isopropanol, gelöst oder dispergiert. Die Lösung/Dispersion wird mit einer Lösung des oben beschriebenen druckempfindlichen Haftklebers in einem geeigneten Gefäß gemischt. Fakultativ, aber nicht zwingend notwendig, können übliche Stoffe wie Füllmittel, Hautschutzstoffe, Klebrigmacher o. ä. zugesetzt werden. Das Gemisch aus Clonidin und dem Acrylat und ggf. weiteren Stoffen wird in einer üblichen Beschichtungsmaschine auf ein Substrat bzw. einen Träger, z. B. aus silikonisierten Kunststoffolien, silikonisiertem Papier o. ä., aufgetragen und in einem nachfolgenden Trockner vom Lösemittel befreit. Nach dem Verlassen des Trockners wird die nun getrocknete und selbstklebende Wirkstoff/Klebstoffmatrix mit einer weiteren Schicht, die z. B. eine Kunststoffolie, ein Vlies, ein Kunststoffschaum, ein Gewebe o. ä. sein kann, zur Abdeckung kaschiert.

In einem weiteren Verarbeitungsschritt werden in einer dem Fachmann bekannten Schneide- oder Stanzvorrichtung die gewünschten Transdermalsysteme mit definierter Form und Größe ausgeschnitten oder gestanzt. Die fertigen Systeme werden zum Schutz in Beutel oder ähnliche Verpackungen eingebracht.

Typischerweise enthalten die Systeme Clonidin im Konzentrationsbereich von 0,1 bis 20 %, bevorzugt im Bereich von 2 bis 10 %. Das Flächengewicht der getrockneten Haftkleberschicht (Matrix) liegt üblicherweise im Bereich von 20 bis 150 g/m², bevorzugt im Bereich von 50 bis 120 g/m². Die Abgaberate liegt im Bereich von 10 bis 1000 µg Clonidin pro Tag, bevorzugt im Bereich von 50 bis 500 µg pro Tag.

Zur Charakterisierung der Transdermalsysteme im Hinblick auf ihre Wirkstoffabgabe werden im wesentlichen zwei Methoden angewendet:
1. In-vitro-Hautpermeationsuntersuchungen
2. In-vitro-Freisetzungsuntersuchungen nach gültigen Pharmakopöen

Hauptpermeationsuntersuchungen werden häufig an isolierter Haut von Nacktmäusen durchgeführt. Dabei wird ein Pflasterstück auf die Oberseite der Haut geklebt und in einer Diffusionszelle montiert. Eine Pufferlösung (Akzeptor) tritt dabei mit der Unterseite der Haut in Kontakt und es wird die zeitabhängige Konzentrationsänderung im Akzeptormedium gemessen.

Die Ergebnisse, die mit den erfindungsgemäßen Zubereitungen erhalten wurden, sind in den folgenden Beispielen aufgeführt.

Die In-vitro-Freisetzungsuntersuchungen werden in Glasgefäßen ausgeführt, die nach den Bestimmungen der Pharmakopöen aufgebaut sind. In einem zylindrischen 1-Liter-Gefäß mit rundem Boden wird das Pflaster auf einer Siebplatte so befestigt, daß die Klebeschicht nach oben weist. Die Siebplatte wird auf den Boden des Gefäßes gebracht und das Gefäß mit Wasser gefüllt, worauf mit einem definierten Rührer zum Konzentrationsausgleich gerührt wird. Hierbei wird ebenfalls die zeitabhängige Konzentration in dem Medium, in das die Freisetzung erfolgt, gemessen. Die Ereignisse dieser Untersuchungen sind in den Beispielen aufgeführt.

Der Unterschied zwischen diesen Methoden besteht darin, daß die Freisetzungsuntersuchungen nur das Freisetzungsverhalten des Wirkstoffes aus dem Pflaster berücksichtigen, was jedoch in der Regel nicht mit der biologischen Wirkung korreliert. Das Hautpermeationsmodell berücksichtigt dagegen zusätzlich zur notwendigen Freisetzung die Verteilung des Wirkstoffes in die Haut und die Diffusion durch die Haut. Hiermit sind in der Regel Korrelationen mit der biologischen Wirkung möglich.

Die folgenden Beispiele veranschaulichen die Erfindung ohne sie zu beschränken.

### Vergleichsbeispiel 1

Ein handelsübliches Clonidin-Pflaster, Catapres® TTS, mit folgender Charakteristik:

| | |
|---|---|
| Gehalt Clonidin | 5 mg |
| Fläche | 7 cm² |

Zusammensetzung (qualitativ):
Mineralöl
Polyisobutylen
kolloidales Siliciumdioxid
mikroporöse Polypropylenmembran
wurde entsprechend dem Europäischen Arzneibuch einer Invitro-Dissolutionsuntersuchung unterzogen. Die Ergebnisse sind in Tabelle 1 dargestellt.

Ergänzend wurde das In-vitro-Hautpermeationsverhalten mit einem Mäusehautmodell untersucht.

### Durchführung:

Ein 1,5 cm² großes Stück Haut von weiblichen Nacktmäusen, das von Unterhautgewebe befreit wurde, wird auf die genau 1 cm² große Öffnung einer automatisierten Diffusionszelle gelegt, mit einem ca. 1,5 cm² großen Stück des Clonidin-Pflasters beklebt und mit einer Andruckvorrichtung auf der Zelle abgedichtet. Dann wird die Zelle mit 25 ml einer physiologischen HEPES-Pufferlösung gefüllt und auf 34° C temperiert. Zu definierten Zeitpunkten werden aus der Pufferlösung Proben gezogen und der Wirkstoffgehalt in ihnen durch Hochdruckflüssigchromatographie bestimmt.

Nach diesem Verfahren wurden alle unten beschriebenen Pflaster untersucht.

Die Ergebnisse sind in Tabelle 2 dargestellt.

### Vergleichsbeispiel 2

Zum Vergleich wurde ein Clonidin-Pflaster unter Verwendung eines selbstvernetzenden Acrylatklebers ohne hinzugefügte Resorptionsförderer hergestellt. Das System hatte folgende Charakteristik:

| | |
|---|---|
| Gehalt Clonidin | 5,25 mg |
| Fläche | 7 cm² |

| Zusammensetzung | |
|---|---|
| Acrylatkleber Duro-Tak 87-2052 | 64,75 mg |
| silikonisierte Polyester-Folie FL2200075 1S** | 7 cm² |
| Polyester-Folie Hostaphan MN 19 MED*** | 7 cm² |
| Der Duro-Tak-Haftkleber wird bei niedriger Temperatur durch den Zusatz von Aluminiumacetylacetonat selbstvernetzend. | |

| | |
|---|---|
| * National Starch & Chemical, NL-Zutphen ** Rexam, NL-Apeldoorn | |
| *** Mitsubishi Polyester Foils, D-Frankfurt | |

### Herstellung:

Clonidin wird in Ethylacetat gelöst. Die Lösung wird einer ausreichenden Menge der handelsüblichen Klebstofflösung zugegeben und mit einem Rührer homogenisiert. Die homogene Lösung wird dann mit einem Ziehrakel auf einen Bogen einer silikonisierten Polyesterfolie (ca. 75 µm) mit definierter Schichtdicke ausgestrichen. Der Bogen wird anschließend zur Trocknung und Vernetzung 30 min bei 50°C in einem Trockenschrank getrocknet. Danach wird eine ca. 19 µm dicke Polyesterfolie auf die klebende Schicht aufkaschiert. Aus dem fertigen Laminat werden mittels einer Handstanze 7 cm² große Pflaster ausgestanzt.

### Hautpermeation:

### s. Vergleichsbeispiel 1

Die Ergebnisse sind in Tabelle 2 dargestellt.

### Vergleichsbeispiel 3

Ein erfindungsgemäßes Clonidin-Pflaster hat folgende Charakteristik:

| | |
|---|---|
| Gehalt Clonidin | 5,25 mg |
| Fläche | 7 cm² |

| Zusammensetzung | |
|---|---|
| Acrylatkleber Duro-Tak 87-4098 | 64,75 mg |
| silikonisierte Polyester-Folie FL200075 1S** | 7 cm² |
| Polyester-Folie Hostaphan MN 19 MED*** | 7 cm² |

### Herstellung:

### s. Vergleichsbeispiel 2

### In-vitro-Freisetzung:

### s. Vergleichsbeispiel 1

Die Ergebnisse sind in Tabelle 1 dargestellt.

### Hautpermeation:

### s. Vergleichsbeispiel 1

Die Ergebnisse sind in Tabelle 2 dargestellt.

**Tabelle 1:**

| In-vitro-Freisetzung Clonidin-Transdermalsystem (Matrixpflaster) | | |
|---|---|---|
| Zeit (Stunden) | Vergl. Bsp. 1 | Beispiel 1 |
| | Freisetzung Clonidin (%) | |
| 2 | 10, 44 | 9,96 |
| 4 | 11,82 | 13,92 |
| 24 | 20,95 | 19,76 |

**Tabelle 2:**

| In-vitro-Hautpermeation Clonidin-Transdermalsystem (Matrixpflaster) | | | |
|---|---|---|---|
| Zeit (Stunden) | Vergl. Bsp. 1 | Vergl. Bsp. 2 | Beispiel |
| | Permeation Clonidin (µg/cm²) | | |
| 3 | 24 | | 24,5 |
| 6 | 56 | | 54 |
| 9 | 80,5 | | 86 |
| 14 | 113,5 | 23,32 | 128,5 |
| 19 | 139 | 46,94 | 165,5 |
| 24 | 163,5 | 55,84 | 186 |
| 32 | | 82,68 | |
| 36 | 233,5 | | 229 |
| 40 | | 105,37 | |
| 48 | 305,5 | | 259,5 |

### Vergleichsbeispiele 4 und 5 und Beispiel 1

### 1. Zusammensetzung der Chargen. die für die Bioäquivalenzstudie verwendet wurden

Beispiele für Zusammensetzung von Pflastern mit Clonidin in nicht vernetztem Acrylatkleber Duro-Tak 87-4098/ Duro-Tak 387-2051 (Fläche 10 cm²):

| *Gehalt:* | | ***5% Clonidin*** | ***5,33% Clonidin*** |
|---|---|---|---|
| Hautabgewandte Seite (Schicht I) | Clonidin | 5,0% | 4,5% |
| | DT 87-4098 | 95,0% | 85,5% |
| | DT 2051 | - | 10,0% |
| *Flächengewicht* | | *70 g*/*m*² | *94g*/*m*^{*2*} |
| | | | |
| | | | |
| Hautzugcwandte Seite (II) | Clonidin | 5,0% | 7,0% |
| | Foral E 105 | 8,5% | 8,5% |
| | DT 87-4098 | 86,5% | 84,5% |
| *Flächengewicht* | | *70 g*/*m*^{*2*} | *46 g*/*m*^{*2*} |
| | | | |

Ausweitbar auf unvernetzten Kleber des Typs *Styren Block Copolymer* mit einem elastomeren mittleren Block, ungesättigt, bestehend aus Polyisopren (SIS, Styren-Isopren-Styren). Ein solches Pflaster hat die Zusammensetzung:

| | | |
|---|---|---|
| Gehalt | Clonidin | 6% |
| | Foral 105E | 5% |
| | Paraffinöl | 5% |
| | Duro-Tak 87-6174 | 84% |
| | Flächengewicht : | 140 g/m²,10 cm² |

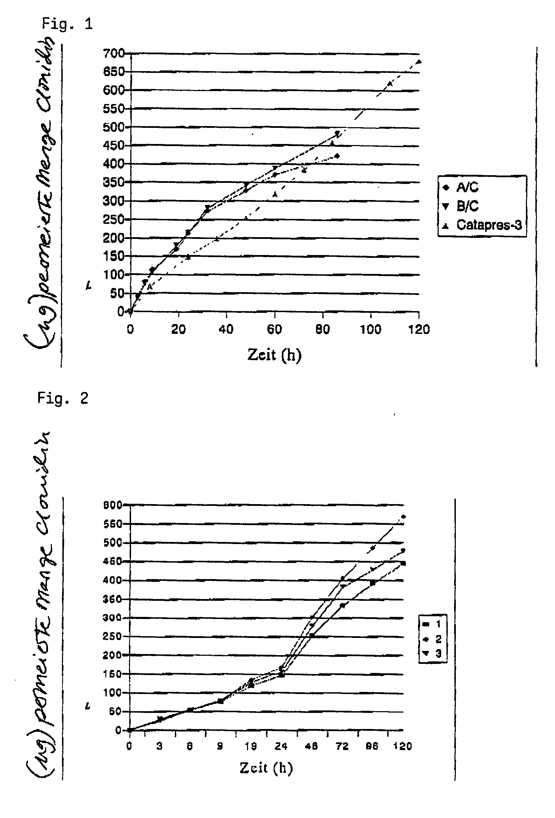

### 2. Hautpermeationsdaten Fig. 1

A. Rezepturen mit unvernetzten Acrylatklebern:
A: Laminatzusammensetzung 4,5% Clonidin in Duro-Tak 4098. Duro-Tak 2051 75:25
B: Laminatzusammensetzung 4,5% Clonidin in Duro-Tak 4098. Duro-Tak 2051 90:10
C: Laminatzusammensetzung 7,1% Clonidin in Duro-Tak 4098 plus 5% Foral 105E
Die Laminate wurden durch Zusammenkaschieren der beiden Laminate gefertigt.

| Zeit (h) | A/C | B/C | Catapres-3 (Vergleichsbeispiel 1) |
|---|---|---|---|
| 0 | 0 | 0 | 0 |
| 3 | 42,9 | 37,01 | |
| 6 | 82,54 | 76,08 | |
| 8 | | | 67,96 |
| 9 | 112,52 | 106,77 | |
| 19 | 168,24 | 178,4 | |
| 24 | 212,06 | 213,73 | 149.03 |
| 32 | 273,45 | 281,98 | |
| 36 | | | 198,47 |
| 48 | 328,01 | 341,37 | 254,41 |
| 60 | 370,78 | 387,99 | 318,22 |
| 72 | | | 383,69 |
| 84 | | | 458,08 |
| 86 | 422,55 | 481,1 | |
| 108 | | | 620,38 |
| 120 | | | 679,48 |

B. Rezepturen mit unvernetztem SIS-Kleber (wie vorne beschrieben) Fig. 2

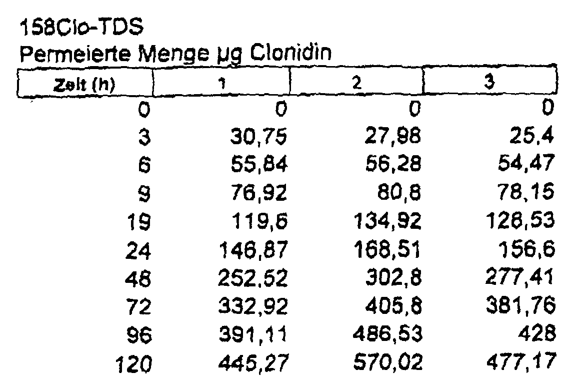

## Patentansprüche

1. Transdermalsystem zur Abgabe von Clonidin aus einer Clonidin enthaltenden Haftkleberschicht aus einem Styrol-Block-Copolymer, die keine toxischen Vernetzer oder Lösemittel oder Resorptionsförderer enthält, sowie neben der Clonidin enthaltenden Haftkleberschicht einer Abdeckung und auf der der Abdeckung gegenüberliegenden Seite einem die Haftkleberschicht temporär abdeckenden und abziehbaren Träger.

2. Transdermalsystem nach Anspruch 1, **dadurch gekennzeichnet, daß** die Haftkleberschicht Clonidin im Konzentrationsbereich von 0,1 bis 20 Gew.-% aufweist.

3. Transdermalsystem nach Anspruch 2, **dadurch gekennzeichnet, daß** die Haftkleberschicht Clonidin im Konzentrationsbereich von 2 bis 10 Gew.-% aufweist.

4. Transdermalsystem nach einem der vorhergehenden Ansprüche, **dadurch gekennzeichnet, daß** die Clonidin enthaltende Haftkleberschicht eine Schicht eines flächigen selbstklebenden Pflasters mit mehrschichtigem Aufbau bildet.

5. Transdermalsystem nach Anspruch 1, **dadurch gekennzeichnet, daß** die Abdeckung aus Kunststoffolie, Kunststoffschaum, Gewebe oder Vlies besteht.

6. Transdermalsystem nach Anspruch 1, **dadurch gekennzeichnet, daß** der Träger aus Kunststoffolie, Papier oder einem Laminat daraus besteht.

7. Transdermalsystem nach Anspruch 6, **dadurch gekennzeichnet, daß** der Träger silikonisiert ist.

8. Transdermalsystem nach Anspruch 5 oder 6, **dadurch gekennzeichnet, daß** es sich bei der Kunststoffolie um Polyester-, Polyethylen- oder Polypropylen-Folie handelt.

9. Transdermalsystem nach einem der Ansprüche 4 bis 8 **dadurch gekennzeichnet, daß** die trockene Haftkleberschicht ein Flächengewicht von 20 bis 150 g/m² hat.

10. Transdermalsystem nach Anspruch 9, **dadurch gekennzeichnet, daß** die trockene Haftkleberschicht ein Flächengewicht von 50 bis 120 g/m² hat.

11. Transdermalsystem nach einem der vorherigen Ansprüche,
**dadurch gekennzeichnet, daß** die Abgaberate 10 bis 1000 µg Clonidin pro Tag beträgt.

12. Transdermalsystem nach Anspruch 11, **dadurch gekennzeichnet, daß** die Abgaberate 50 bis 500 µg Clonidin pro Tag beträgt.

13. Transdermalsystem nach einem der vorhergehenden Ansprüche, **gekennzeichnet durch** ein Styrol-Isopren-Styrol-Copolymer (Styren-Isopren-Styren-Copolymer) als Styrol-Block-Copolymer (Styren-Block-Copolymer).

14. Transdermalsystems nach einem der vorherigen Ansprüche zur Behandlung von Hypertonie, Migräne, Angstzuständen, hyperkinetischen Verhaltensstörungen, Entzugserscheinungen bei Alkohol- oder Drogenentzug und menopausalen Symptomen.

## Claims

1. A transdermal system for the release of clonidine from a clonidine-containing adhesive layer on the basis of a styrene block copolymer, which does not contain toxic crosslinkers or solvents or resorption promoters, as well as, in addition to the clonidine-containing adhesive layer, a cover layer and, on the side facing away from the cover layer, a carrier layer which is removable and which temporarily covers the adhesive layer.

2. The transdermal system in accordance with Claim 1, **characterized in that** the adhesive layer contains clonidine in a concentration ranging from 0.1 to 20 percent by weight.

3. The transdermal system in accordance with Claim 2, **characterized in that** the adhesive layer contains clonidine in a concentration ranging from 2 to 10 percent by weight.

4. The transdermal system in accordance with at least one of the preceding Claims, **characterized in that** the adhesive layer containing clonidine forms one layer of a flat, self-adhesive band with a multilayer structure.

5. The transdermal system in accordance with Claim 1, **characterized in that** the cover layer is made from plastic foil, plastic foam, woven fabric, or fleece.

6. The transdermal system in accordance with Claim 1, **characterized in that** the carrier layer is made from plastic foil, paper, or a laminate thereof.

7. The transdermal system in accordance with Claim 6, **characterized in that** the carrier layer is siliconized.

8. The transdermal system in accordance with at least one of Claim 5 or 6, **characterized in that** plastic foil is a polyester, polyethylene, or polypropylene foil.

9. The transdermal system in accordance with at least one of Claims 4 through 8, **characterized in that** the dry adhesive layer has an areal weight of between 20 and 150 g/m².

10. The transdermal system in accordance with Claim 9, **characterized in that** the dry adhesive layer has an areal weight of between 50 and 120 g/m².

11. The transdermal system in accordance with at least one of the preceding Claims, **characterized in that** the release rate is between 10 and 1000 µg of clonidine per day.

12. The transdermal system in accordance with Claim 11, **characterized in that** the release rate is between 50 and 500 µg of clonidine per day.

13. The transdermal system in accordance with at least one of the preceding Claims, **characterized by** a styrene-isoprene-styrene copolymer (styrene-isoprene-styrene copolymer) as styrene block copolymer (styrene block copolymer).

14. The transdermal system in accordance with at least one of the preceding Claims for the treatment of hypertonia, migraines, anxieties, hyperkinetic behavioral disorders, alcohol or drug-related withdrawal symptoms, and menopausal symptoms.

## Revendications

1. Système transdermique pour l'administration de clonidine à partir d'une couche adhérente contenant la clonidine en un copolymère séquencé de styrène, qui ne contient aucun agent de réticulation ou solvant ou accélérateur de résorption toxique, ainsi qu'en plus de la couche adhérente contenant la clonidine, une couche de couverture et sur la face opposée à la couche de couverture, un support couvrant temporairement la couche adhérente et pouvant être éliminé.

2. Système transdermique selon la revendication 1, **caractérisé en ce que** la couche adhérente présente la clonidine dans un intervalle de concentration allant de 0,1 à 20 % en poids.

3. Système transdermique selon la revendication 2, **caractérisé en ce que** la couche adhérente présente la clonidine dans un intervalle de concentration allant de 2 à 10 % en poids.

4. Système transdermique selon l'une quelconque des revendications précédentes, **caractérisé en ce que** la couche adhérente contenant la clonidine forme une couche d'un timbre auto-collant plat avec une structure multicouche.

5. Système transdermique selon la revendication 1, **caractérisé en ce que** la couche de couverture consiste en une feuille plastique, une mousse plastique, un tissu ou un voile.

6. Système transdermique selon la revendication 1, **caractérisé en ce que** le support consiste en une feuille plastique, un papier ou un stratifié.

7. Système transdermique selon la revendication 6, **caractérisé en ce que** le support est siliconé.

8. Système transdermique selon la revendication 5 ou 6, **caractérisé en ce que** la feuille plastique consiste en une feuille de polyester, de polyéthylène ou de polypropylène.

9. Système transdermique selon l'une quelconque des revendications 4 à 8, **caractérisé en ce que** la couche adhérente sèche a un poids surfacique allant de 20 à 150 g/m².

10. Système transdermique selon la revendication 9, **caractérisé en ce que** la couche adhérente sèche a un poids surfacique allant de 50 à 120 g/m².

11. Système transdermique selon l'une quelconque des revendications précédentes, **caractérisé en ce que** le taux d'administration se situe dans l'intervalle allant de 10 à 1000 µg de clonidine par jour.

12. Système transdermique selon la revendication 11, **caractérisé en ce que** le taux d'administration se situe dans l'intervalle allant de 50 à 500 µg de clonidine par jour.

13. Système transdermique selon l'une quelconque des revendications précédentes, **caractérisé par** un copolymère styrène-isoprène-styrène (styren-isopren-styren copolymer) comme copolymère séquencé de styrène (styren-block-copolymer).

14. Système transdermique selon l'une quelconque des revendications précédentes, pour le traitement de l'hypertonie, des migraines, des états anxieux, des troubles de comportement hyperkinétiques, des phénomènes de désaccoutumance en cas de désintoxication d'alcool ou de drogue et des symptômes de la ménopause.
